# EUROPEAN PATENT APPLICATION

(11) **EP 4 681 658 A1**
(43) Date of publication of application: **21.01.2026**
(21) Application number: 25188509.1
(22) Date of filing: 09.07.2025
(51) Int. Cl.: A61B 17/12

(54) **DOUBLE SEALING LEFT ATRIAL APPENDAGE OCCLUDER WITH INTERCHANGEABLE COMPONENTS**

(30) Priority: 15.07.2024 US 202463671459 P
(71) Applicant: St. Jude Medical, Cardiology Division, Inc., St. Paul, MN 55117 (US)
(72) Inventor: MEYER, Michael P., Minnetrista, 55359 (US); ERZBERGER, Gary, Corcoran, 55340 (US)
(74) Representative: Gill Jennings & Every LLP

(57) **Abstract**

A kit for assembling a collapsible and expandable occluder (156dc) for occluding a left atrial appendage (LAA) may include a plurality of proximal discs (156a-c) and a plurality of distal lobes (158a-e). Each proximal disc may be configured to cover an ostium of the LAA in an implanted condition of the occluder, and each of the plurality of proximal discs may have a different diameter in an expanded condition than other ones of the plurality of proximal discs. Each distal lobe may be configured to be received within a cavity of the LAA in the implanted condition of the occluder, and each of the plurality of distal lobes may have a different diameter in an expanded condition than other ones of the plurality of proximal discs. Each of the plurality of proximal discs may be configured to couple to any one of the plurality of distal lobes to form the occluder.

## Description

### Cross-Reference to Related Applications

This application claims the benefit of the filing date of U.S. Provisional Patent Application No. 63/671,459, filed July 15, 2024, the disclosure of which is hereby incorporated by reference herein.

### Field of the Disclosure

The present disclosure relates generally to medical devices that are used in the human body. In particular, the present disclosure is directed to an occlusion device having a configuration that allows for more consistent and stable anchoring and sealing of the occlusion device within a tissue cavity. More specifically, the present disclosure is directed to an occlusion device with features to provide for a double sealing of a target site while including interchangeable components to optimize the ability to appropriately size the devices for different patients.

### Background

An occluder is a medical device used to treat (*e.g.,* occlude) tissue at a target site within the human body, such as an abnormality, a vessel, an organ, an opening, a chamber, a channel, a hole, a cavity, a lumen, or the like. For example, an occluder may be used for Left Atrial Appendage ("LAA") closures. An LAA is a normal anatomical structure in which there is a sac in the muscle wall of the left atrium. When a patient experiences atrial fibrillation ("AFib"), a blood clot may be formed within the LAA which may become dislodged and enter into the blood stream. By occluding the LAA, the release of blood clots from the LAA may be significantly reduced, if not eliminated. Various techniques have been developed to occlude the LAA. For instance, balloon-like devices have been developed that are configured to be implanted completely within the cavity of the LAA, while surgical techniques have also been developed where the cavity of the LAA is inverted and surgically closed.

Despite these techniques, it would be advantageous to provide an improved occlusion device that offers enhanced sizing options while maintaining a double sealing functionality.

### Summary of the Disclosure

According to a first aspect of the disclosure, a kit for assembling a collapsible and expandable occluder for occluding a left atrial appendage ("LAA") includes a plurality of proximal discs and a plurality of distal lobes. Each proximal disc may be configured to cover an ostium of the LAA in an implanted condition of the occluder, and each of the plurality of proximal discs may have a different diameter in an expanded condition than other ones of the plurality of proximal discs. Each distal lobe may be configured to be received within a cavity of the LAA in the implanted condition of the occluder, and each of the plurality of distal lobes may have a different diameter in an expanded condition than other ones of the plurality of distal lobes. Each of the plurality of proximal discs may be configured to couple to any one of the plurality of distal lobes to form the occluder. Each of the plurality of proximal discs may be formed of a braided mesh and each of the plurality of distal lobes may be formed as a braided mesh. Each of the plurality of proximal discs may be formed of overlapping wire petals and each of the plurality of distal lobes may be formed of a laser cut tube of shape memory material. Each of the plurality of proximal discs may have a corresponding first connection portion coupled thereto, and each of the plurality of distal lobes may have a corresponding second connection portion coupled thereto, each of the first connection portions being configured to couple to any one of the second connection portions. Each first connection portion may include a shaft and an enlarged head, and each second connection portion may include a receiver defining a channel therethrough, the shaft and enlarged head configured to pass through the channel in a first direction, the enlarged head being compressible and having a diameter that is larger than a diameter of the channel when the enlarged head is in an uncompressed condition. Each of the first connection portions may be configured to couple to any one of the second connection portions to form an assembled connector, the assembled connector having a variable length. Each first connection portion may include a ratchet pawl, and each second connection portion may include a plurality of serrations so that each second connection portion is configured to pass through any one of the first connection portions in a first direction, but not in a second direction opposite the first direction.

In some examples, a plurality of connectors may be configured to couple any of the plurality of proximal discs to any one of the plurality of distal lobes, each of the plurality of connectors having a different length. The plurality of connections may be provided as part of the kit. Each of the plurality of connectors may be a spring connector having a first end, a second end, and a main spring body between the first end and the second end, the main spring body of each of the plurality of connectors having a different length. The plurality of proximal discs may each be substantially circular with a connection portion extending from a radial center thereof, and the kit may further include at least one eccentric proximal disc having a connection portion extending therefrom, the connection portion of the at least one eccentric proximal disc being offset from a radial center of the at least one eccentric proximal disc. The plurality of proximal discs may include at least one circular proximal disc and at least one oval-shaped proximal disc. The kit may include a single container that includes therein the plurality of proximal discs and the plurality of distal lobes. Otherwise, the kit may include a first container that includes therein the plurality of proximal discs, and a second container that includes therein the plurality of distal lobes. In other examples, the kit may include a plurality of first containers that include therein the plurality of proximal discs, and a plurality of second containers that include therein the plurality of distal lobes.

According to another aspect of the disclosure, a method of occluding a left atrial appendage ("LAA") of a patient with an occluder includes imaging the LAA of the patient and performing at least one measurement of the LAA of the patient based on the imaging. Based on the at least one performed measurement, a proximal disc may be selected from a plurality of proximal discs, and a distal lobe may be selected from a plurality of distal lobes. The selected proximal disc may be coupled to the selected distal lobe to assemble the occluder. The assembled occluder may be attached to a delivery system and the assembled occluder may be advanced through a delivery sheath toward the LAA while the assembled occluder is in a collapsed condition. The assembled occluder may be deployed into the LAA of the patient by expanding the assembled occluder from the collapsed condition to an expanded condition. Performing the at least one measurement of the LAA may include measuring one or more of: a depth of the LAA, a diameter of the LAA, a size of an ostium of the LAA, a shape of the ostium of the LAA, a distance between the ostium of the LAA and a mitral valve of the patient, and a distance between the ostium of the LAA and a pulmonary ridge of the patient. Performing the at least one measurement of the LAA may include measuring the diameter of the LAA, and the distal lobe may be selected from the plurality of distal lobes based on the measured diameter of the LAA. Performing the at least one measurement of the LAA may include measuring the size of the ostium of the LAA, and the proximal disc may be selected from the plurality of proximal discs based on the measured size of the ostium of the LAA. Coupling the selected proximal disc to the selected distal lobe may include coupling a first connection portion of the selected proximal disc to a second connection portion of the selected distal lobe. Each of the plurality of proximal discs may have a different diameter when expanded than other ones of the plurality of proximal discs, and each of the plurality of distal lobes may have a different diameter when expanded than other ones of the plurality of distal lobes. Based on the at least one performed measurement, a connector may be selected from a plurality connectors, and after selecting the connector, a first end of the selected connector may be coupled to the selected proximal disc, and a second end of the selected connector may be coupled to the selected distal lobe to assemble the occluder. Performing the at least one measurement of the LAA may include measuring a distance between an ostium of the LAA and a pulmonary ridge of the patient, and the connector may be selected from the plurality connectors based on the measured distance.

According to another example of the disclosure, a method of assembling a left atrial appendage occluder ("LAAO") may include selecting a proximal disc from a plurality of proximal discs and selecting a distal lobe from a plurality of distal lobes. The selected proximal disc may be coupled to the selected distal lobe to assemble the LAAO. Each of the plurality of proximal discs may have a different diameter in an expanded condition than other ones of the plurality of proximal discs. Each of the plurality of distal lobes may have a different diameter in an expanded condition than other ones of the plurality of distal lobes. The selected proximal disc may be configured to cover an ostium of a left atrial appendage ("LAA") of a patient, and the selected distal lobe may be configured to be received within a cavity of the LAA of the patient. Each of the plurality of proximal discs may be configured to couple to any one of the plurality of distal lobes to form the LAAO. The method may include imaging the LAA of the patient, and performing at least one measurement of the LAA of the patient based on the imaging. One or both steps of (i) selecting the proximal disc from the plurality of proximal discs and (ii) selecting the distal lobe from the plurality of distal lobes may be performed based on the at least one performed measurement. Performing the at least one measurement of the LAA may include measuring one or more of: a depth of the LAA, a diameter of the LAA, a size of the ostium of the LAA, a shape of the ostium of the LAA, a distance between the ostium of the LAA and a mitral valve of the patient, and a distance between the ostium of the LAA and a pulmonary ridge of the patient. Performing the at least one measurement of the LAA may include measuring the diameter of the LAA, and selecting the distal lobe from the plurality of distal lobes may be performed based on the measured diameter of the LAA. Performing the at least one measurement of the LAA may include measuring the size of the ostium of the LAA, and selecting the proximal disc from the plurality of proximal discs may be performed based on the measured size of the ostium of the LAA.

According to a further example of the disclosure, an occluder for occluding a left atrial appendage ("LAA") includes a modular proximal disc, the modular proximal disc configured to cover an ostium of the LAA in an implanted condition of the occluder, and a modular distal lobe, the modular distal lobe configured to be received within a cavity of the LAA in the implanted condition of the occluder. The modular proximal disc may have a first connection portion, and the modular distal lobe may have a second connection portion, the first connection portion being configured to couple to the second connection portion to assemble the occluder. The first connection portion may be configured to couple to the second connection portion via a separate connector. The modular proximal disc may be selected from a group of modular proximal discs, and the modular distal lobe may be selected from a group of modular distal lobes. Each one of the group of modular proximal discs may have a diameter, when expanded, that is different than an expanded diameter of each of the other ones of the group of modular proximal discs. Each one of the group of modular distal lobes may have a diameter, when expanded, that is different than an expanded diameter of each of the other ones of the group of modular distal lobes. Each one of the group of modular proximal discs may be configured to couple to any one of the group of modular distal lobes. The modular proximal disc may be formed with a first base design, and the modular distal lobe may be formed with a second base design different from the first base design so that the proximal disc is more flexible than the distal lobe, and the distal lobe is stiffer than the proximal disc.

### Brief Description of the Drawings

Fig. 1 illustrates an occluder according to the prior art.
Fig. 2 is a schematic diagram of a delivery system in accordance with the present disclosure.
Fig. 3 illustrates the medical device of Fig. 1 being deployed into a left atrial appendage.
Figs. 4A-4B are front and side illustrations, respectively, of a particular size of the occluder of Fig. 1 having been implanted into a patient's LAA with a resulting gap.
Fig. 5 is a side illustration of a particular size of the occluder of Fig. 1 having been implanted into a patient's LAA which as a highly conical shape.
Fig. 6 is a side illustration of a particular size of the occluder of Fig. 1 having been implanted into a patient's LAA which has a relatively large pulmonary ridge.
Fig. 7 is a side illustration of a particular size of the occluder of Fig. 1 having been implanted into a patient's LAA which has multi-branched anatomical variation.
Fig. 8 is a side illustration of a particular size of the occluder of Fig. 1 having been implanted into a patient's LAA in which the disc impinges on the mitral valve.
Fig. 9 is a side illustration of a particular size of the occluder of Fig. 1 having been implanted into a patient's LAA in which the disc extends into the left atrium.
Fig. 10 is a side illustration of an occluder, according to an aspect of the disclosure, having a relatively small lobe connected to a relatively large disc implanted into a patient's LAA.
Fig. 11 shows an example kit of differently sized discs that may be mixed and matched with differently sized lobes to configure an occluder with unique size combinations of the disc and lobe.
Figs. 12A-B illustrate selected sizes of a lobe and a disc of a kit prior to being coupled, and after being coupled to form an occluder, respectively.
Figs. 13A-B illustrate exemplary individual disc and lobe components, respectively, of a kit of components formed as braided mesh components.
Figs. 14A-B illustrate exemplary individual disc and lobe components, respectively, of a kit of components formed without braiding.
Figs. 15A-B illustrate two connector components according to an aspect of the disclosure.
Fig. 15C illustrates the two connector components of Figs. 15A-B coupled together.
Fig. 16 illustrates an example of a variable length connector for connecting a disc to a lobe.
Fig. 17 illustrates an example of an occluder kit that includes multiple sizes of connectors for coupling a disc to a lobe.
Fig. 18 shows one example of a spring connector that may be provided in multiple sizes as part of a kit with a plurality of discs and lobes.
Fig. 19 shows one example of a suture connector that may be provided in multiple sizes as part of a kit with a plurality of discs and lobes.
Figs. 20A-B show one example of a clasp connector in closed and opened conditions, respectively, that may be provided in multiple sizes as part of a kit with a plurality of discs and lobes.
Figs. 21A-B illustrate selected sizes of an eccentric lobe and a disc of a kit prior to being coupled, and after being coupled to form an occluder, respectively.

### Detailed Description of the Disclosure

The present disclosure relates generally to medical devices that are used in the human body. Specifically, the present disclosure provides medical devices including occlusion devices having features for enhancing the ability to appropriately size an occluder for a particular patient while maintaining benefits of double sealing of the target site. The disclosed embodiments may lead to more consistent and improved patient outcomes. It is contemplated, however, that the described features and methods of the present disclosure as described herein may be incorporated into any number of systems as would be appreciated by one of ordinary skill in the art based on the disclosure herein.

Although the exemplary embodiments of the medical devices are described as treating a target site including a LAA, it is understood that the use of the term "target site" is not meant to be limiting, as the medical device may be configured to treat any target site, such as an abnormality, a vessel, an organ, an opening, a chamber, a channel, a hole, a cavity, or the like, located anywhere in the body. The term "vascular abnormality," as used herein is not meant to be limiting, as the medical device may be configured to bridge or otherwise support a variety of vascular abnormalities. For example, the vascular abnormality could be any abnormality that affects the shape of the native lumen, such as an atrial septal defect, a lesion, a vessel dissection, or a tumor.. Furthermore, the term "lumen" is also not meant to be limiting, as the vascular abnormality may reside in a variety of locations within the vasculature, such as a vessel, an artery, a vein, a passageway, an organ, a cavity, or the like. As used herein, the term "proximal" refers to a part of the medical device or the delivery device that is closest to the operator, and the term "distal" refers to a part of the medical device or the delivery device that is farther from the operator at any given time as the medical device is being delivered through the delivery device. In addition, the terms "deployed" and "implanted" may be used interchangeably herein.

Some embodiments of the present disclosure provide an improved percutaneous catheter directed intravascular occlusion device for use in the vasculature in patients' bodies, such as blood vessels, channels, lumens, a hole through tissue, cavities, and the like, such as a LAA. Other physiologic conditions in the body occur where it is also desirous to occlude a vessel or other passageway to prevent blood flow into or therethrough. These device embodiments may be used anywhere in the vasculature where the anatomical conditions are appropriate for the design.

The medical device may include one or more layers of occlusive material, wherein each layer may be comprised of any material that is configured to substantially preclude or occlude the flow of blood so as to facilitate thrombosis. As used herein, "substantially preclude or occlude flow" shall mean, functionally, that blood flow may occur for a short time, but that the body's clotting mechanism or protein or other body deposits on the occlusive material results in occlusion or flow stoppage after this initial time period.

Some embodiments of the present disclosure may be formed by a plurality of wire strands having a predetermined relative orientation with respect to one another. However, it is understood that according to additional embodiments of the present disclosure, that the medical device could be etched or laser cut from a tube, or the device could comprise an occlusion material coupled to a scaffolding structure or a plurality of slices of a tubular member coupled together.

The present disclosure now will be described more fully hereinafter with reference to the accompanying drawings, in which some, but not all embodiments of the disclosure are shown. Indeed, this disclosure may be embodied in many different forms and should not be construed as limited to the embodiments set forth herein; rather, these embodiments are provided so that this disclosure will satisfy applicable legal requirements. Like numbers refer to like elements throughout.

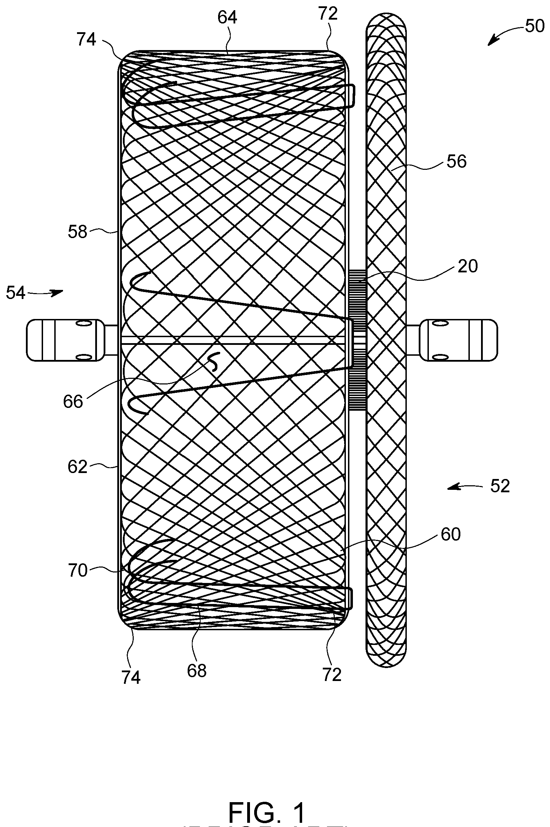

In at least some conventional or known medical devices used for the occlusion of abnormalities, such as a medical device 50 (also referred to as occluder 50 herein) shown in Fig. 1, occluder 50 includes a proximal end 52 and a distal end 54, with a disc 56 at proximal end 52 and a lobe 58 at distal end 54. The lobe 58 has a proximal edge 60 (also referred to as a proximal face), a distal edge 62 (also referred to as a distal face), and a middle or central portion 64 that defines a cavity 66. The disc 56 may be connected to the lobe 58 via a relatively small diameter connecting portion or waist 20, which may be integrally formed with both the disc 56 and the lobe 58. The occluder 50 also includes stabilizing anchors 68 secured to a radially outer or circumferential surface of middle portion 64. The stabilizing anchors 68 terminate in a hook 70 at free ends thereof, and thereby facilitate retention of the occluder 50 at a target site and preventing the occluder 50 from becoming dislodged from the target site after deployment.

In this known occluder 50, proximal edge 60 and distal edge 62 adjoin middle portion 64 at a first relatively blunt or sharp (*e.g*., non-rounded) transition 72 and a second blunt transition 74, respectively. First blunt transition 72 connects proximal edge 60 to middle portion 64 by an approximately 90 degree angle. Likewise, second blunt transition 74 connects distal edge 62 to middle portion 64 by an approximately 90 degree angle. First blunt transition 72 and second blunt transition 74 partially define a generally rectangular cross section to lobe 58, leading to relatively blunt circumferential edges of the device and relatively high radial force applied to the surrounding tissue. In the illustrated embodiment, the occluder 50 may be formed by a single or multiple layers of mesh formed by braiding together one or more strands of wire, which for example may be nitinol wires or other wires formed of shape-memory material. The ends of the braided wires may be secured via any suitable means, for example including end caps and/or end screws. In some embodiments, the occluder 50 may be shape set (for example via heat treatment) to have a shape similar to that shown in Fig. 1 in the absence of applied forces, with the occluder 50 being collapsible to a small diameter for delivery through the vasculature within a catheter, with the occluder 50 returning (or attempting to return) to its set shape during deployment into the target site, for example the LAA. In some embodiments, one or more occluding fabrics may be provided on or in the disc 56 and/or lobe 58 to enhance sealing and occlusion upon deployment. In some embodiments, the lobe 58 of the occluder 50 may be generally cylindrical (*e.g*. with a circular or substantially circular cross section) and the disc 56 of occluder 50 may similarly be generally cylindrical or disc-shaped (*e.g*. with a circular or substantially circular cross section).

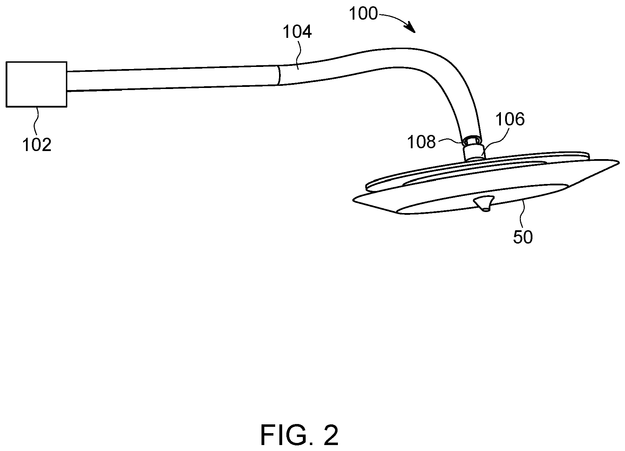

Turning now to Fig. 2, a schematic diagram of a delivery system 100 is shown. Delivery system 100 includes a delivery device 102 including a catheter 104 and a coupling member 106 configured to couple a distal end of a delivery cable 108 to a medical device 50 (which may be any of the occluders described herein) for facilitating the deployment of medical device 50 at a target site. Medical device 50 is deployed to treat the target site, and, in the example embodiment, is an occlusion device ("occluder").

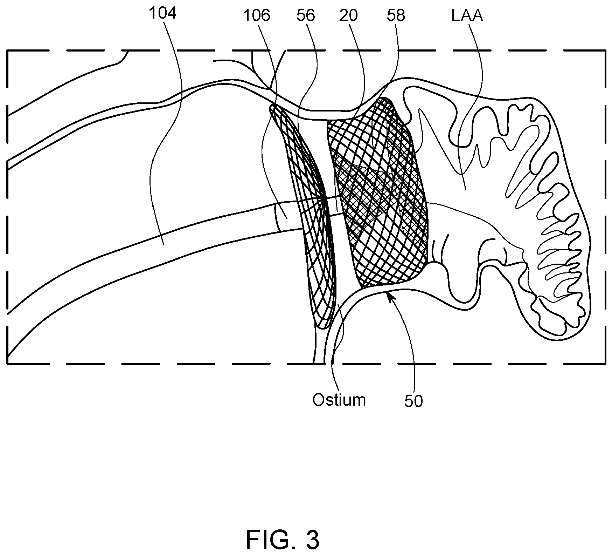

Fig. 3 shows occluder 50 having been deployed from catheter 104 into the LAA prior to decoupling the catheter 104 from the occluder 50. Compared to other known LAA occluders, occluder 50 effectively creates a double-seal when deployed into the LAA. First, the lobe 58 is positioned within the neck of the LAA and is generally capable of closely conforming to the shape of the tissue forming the neck of the LAA, sealing the majority of the LAA from the left atrium. The position of the lobe 58 may be secured, at least in part, via engagement between stabilizing anchors 68 and tissue forming the LAA. Second, the disc 56 is configured to be positioned in contact with the ostium of the LAA (*e.g.,* the orifice leading from the left atrium into the LAA). The waist 20 generally is under some amount of tension which results in the disc 56 being pulled against the ostium of the LAA, and a second seal is formed by the disc 56 covering the opening leading into the LAA. This double sealing functionality is desirable because it may provide better and/or redundant sealing compared to other LAA occluders that have only a single sealing functionality, and also because it may allow for more anatomies (LAA shapes are highly variable) to be treated compared to other LAA occlusion devices. While this double-sealing functionality may be highly desirable, it may also result in some limitations. For example, occluder 50 is typically offered in a variety of sizes. For example, a total of eight sizes of occluder may be offered in some configurations, including four "small" sizes in which the lobe 58 has an axial length of about 7.5 mm, and four "large" sizes in which the lobe 58 has an axial length of about 10 mm. The four small sizes may include lobes 58 having a diameter of about 16 mm, about 18 mm, about 20 mm, and about 22 mm, with the disc 56 having a diameter of about 22 mm, about 24 mm, about 26 mm, and about 28 mm, respectively. The four large sizes may include lobes 58 having a diameter of about 25 mm, about 28 mm, about 31 mm, and about 34 mm, with the disc 56 having a diameter of about 32 mm, about 35 mm, about 38 mm, and about 41 mm, respectively. Although these size options may be suitable to treat a large number of patients, practical limitations prevent the occluder 50 from being offered in significantly more size options and combinations between the lobe 58 and the disc 56. For example, if it were desirable to have any of the different eight sizes of disc 56 available for combination with any of the different eight sizes of lobe 58, sixty-four separate devices would need to be produced, which would not be practical. If additional options, such as differently shaped discs (*e.g.* oval or circular options) or additional lengths of the connecting portion or waist, are also desired, the total number of options for combinations of shapes and sizes for occluder 50 may become nearly endless. It should also be noted that available shelf space is at a premium in most catheter labs ("cath labs"). Thus, even if dozens or hundreds of size combinations of occluder 50 could be produced by a manufacturer and all of the inventory was able to be managed without undue burden, there might simply not be shelf space to have the desired number of combination sizes available within the cath lab due to the small available shelf space within most cath labs.

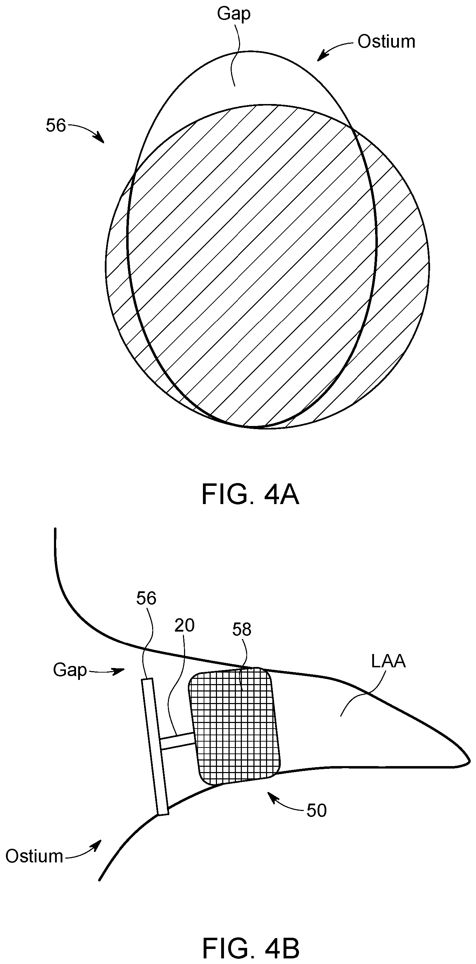

There are various situations in which the standard available eight sizes of occluder 50 may result in the disc 56 being too small (for the given size of lobe 58) to occlude the LAA in a fully desirable manner. Figs. 4-7 provide examples of this situation. At least some of the undesirable situations shown in Figs. 4-7 are the result of the disc 56 and lobe 58 having corresponding sizes (*e.g.,* larger lobes paired with larger discs and smaller lobes paired with smaller discs).

Figs. 4A and 4B are front (*e.g.,* looking from the left atrium toward the LAA) and side illustrations of a particular size of occluder 50 having been implanted into a patient's LAA. As best shown in Fig. 4A, the ostium of the LAA has an oval or eccentric shape. The size of the occluder 50 selected for implantation into the LAA may traditionally be based on the size of the lobe 58 to ensure the lobe 58 can fit within and seal against the LAA. However, in situations in which the ostium of the LAA is highly oval or eccentric, for a given size of the lobe 58 that is appropriate for the LAA, the corresponding size of the disc 56 may often be too small. For example, as shown in Figs. 4A-B, the round or circular disc 56 is able to cover part, but not all, of the eccentric ostium, resulting in a gap that may allow blood to flow into and out of the LAA, as well as potentially allowing blood to stagnate and for thrombus to form on the occluder 50 at undesirable locations where they can embolize and cause stroke. At least some embodiments described in greater detail below may help avoid the situation shown in Figs. 4A-B, for example by being able to pair a larger circular disc with the particular desired size of the lobe, and/or by being able to pair an oval or eccentric disc with the particular desired size of the lobe.

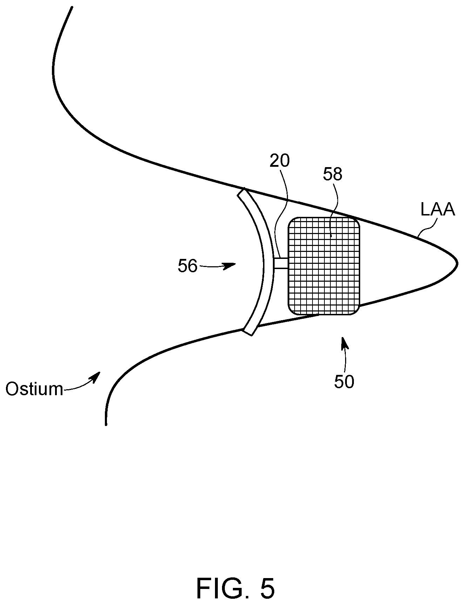

Fig. 5 is a side illustration of a particular size of occluder 50 having been implanted into a patient's LAA. In this example, the patient's LAA has a highly conical shape. At least in part due to the conical shape of the LAA, a relatively small lobe 58 may be needed for occluder 50 to appropriately anchor and seal within the LAA. However, as explained above, an occluder 50 with a relatively small lobe 58 will typically also have a relatively small disc 56. As shown in Fig. 5, the result is that the disc 56 does not satisfactorily cover the ostium of the LAA, meaning that blood may still be able to flow into the LAA and stagnate, with the possible result of thrombi forming in the LAA and embolizing to possibly cause a stroke. At least some embodiments described in greater detail below may help avoid the situation shown in Fig. 5, for example by being able to pair a larger disc with the relatively small sized lobe shown in Fig. 5, and/or by being able to pair a longer connector 20 between the lobe 58 and disc 56 of Fig. 5.

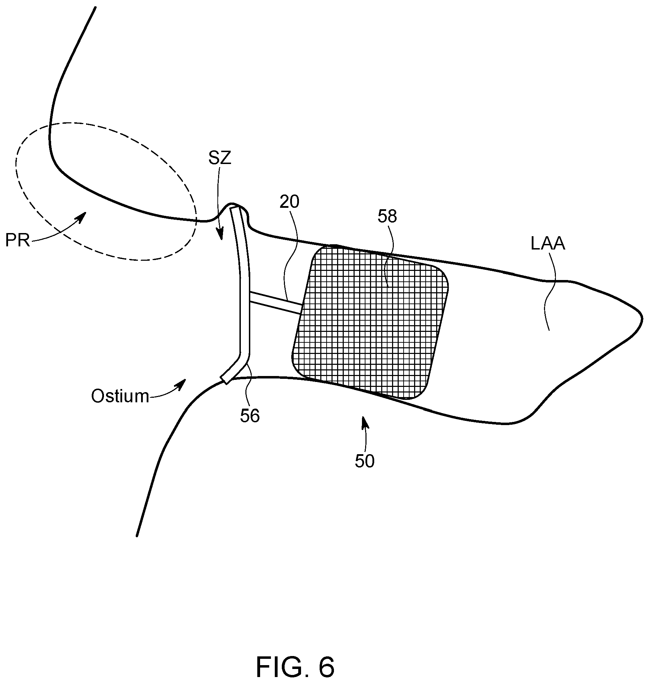

Fig. 6 is a side illustration of a particular size of occluder 50 having been implanted into a patient's LAA. In this example, the patient's LAA has a relatively large pulmonary ridge PR. One of the goals of the double-sealing style occluder 50, in addition to having the lobe 58 seal within the LAA, is to have the disc 56 fully cover the ostium of the LAA, which typically includes having the disc 56 cover the pulmonary ridge PR and having a generally smooth transition between the proximal surface of the disc 56 and the tissue forming and/or surrounding the LAA. In the situation of Fig. 6, due to the relatively large size of the pulmonary ridge PR, the disc 56 stops short of being able to cover pulmonary ridge PR. In the illustrated situation, this results in the disc 56 pressing into (*e.g*., tenting) the tissue near the ostium of the LAA, which may create a harsh or acute (rather than smooth) transition between the disc 56 and the tissue, creating a stagnation zone SZ where blood may stagnate and be at high risk of thrombus formation. At least some embodiments described in greater detail below may help avoid the situation shown in Fig. 6, for example by being able to pair a relatively large disc with the lobe 58 shown in Fig. 6, and/or by being able to pair a longer connector 20 between the lobe 58 and disc 56 of Fig. 6. With such a configuration, for the given size of lobe 58 needed for proper anchoring and sealing within the LAA, the disc 56 may be able to cover the pulmonary ridge PR and/or form a smooth transition between the disc 56 and tissue to minimize or avoid stagnation zones SZ of the type shown in Fig. 6.

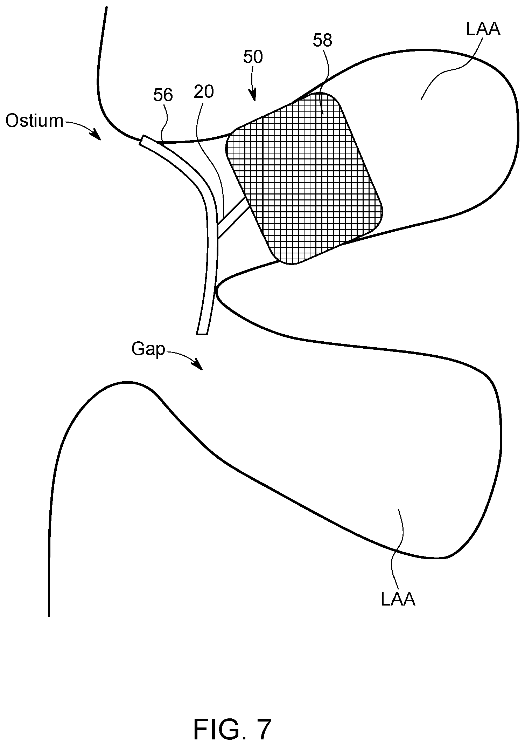

Fig. 7 is a side illustration of a particular size of occluder 50 having been implanted into a patient's LAA. However, as noted above, LAA anatomies between patients may be highly variable. In the particular anatomy shown in Fig. 7, the patient has a multi-branched LAA, which effectively includes an LAA with a single ostium leading to two separate lobes or branches of the LAA. In order to properly anchor the occluder 50 into the LAA, a relatively small lobe 58 may need to be used to fit within either one of the two lobes or branches of the LAA. However, as noted above, occluders 50 with a relatively small lobe 58 generally have a relatively small disc 56. As a result, in the situation of Fig. 7, although the lobe 58 is properly anchored within one of the two branches or lobes of the LAA, the relatively small disc 56 is unable to fully cover the ostium leading to both branches of the LAA. This situation leads to a gap in the sealing, and thus fails to fully occlude the LAA which is the intent of the procedure. At least some embodiments described in greater detail below may help avoid the situation shown in Fig. 7, for example by being able to pair a relatively large disc with the lobe 58 shown in Fig. 7. With such a configuration, for the given size of lobe 58 needed for proper anchoring and sealing within one of the branches or lobes of the LAA, the disc 56 may be able to cover ostium that leads to the two branches of the LAA, eliminating the gap shown in Fig. 7.

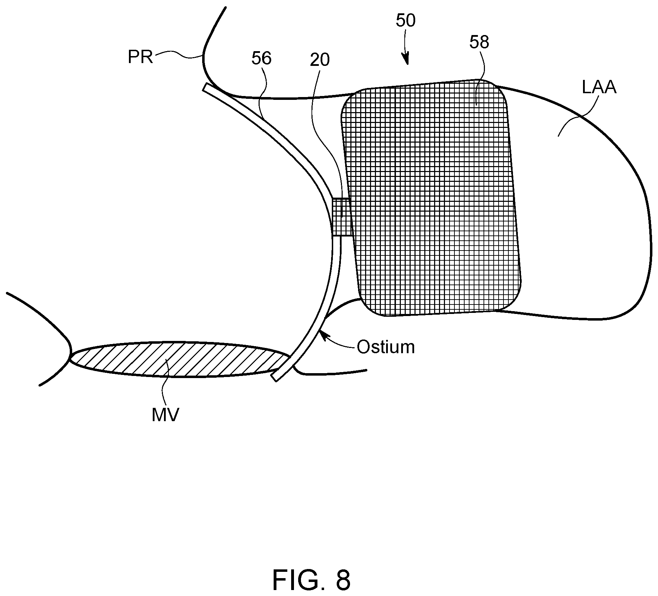

As noted above, Figs. 4-7 illustrate examples of situations in which the standard type of sizing of occluder 50 results in the disc 56 being too small, for the given size of lobe 58, to occlude the LAA in a fully desirable manner. However, the opposite situation may also occur when using standard sizing types of occluders 50, in which the disc 56 is too larger for the given size of lobe 58. At least some undesirable situations shown in Figs. 8-9, in which the disc 56 is too large for the given size of the lobe 58, are the result of the disc 56 and lobe 58 having corresponding sizes (*e.g.,* larger lobes paired with larger discs and smaller lobes paired with smaller discs).

Fig. 8 is a side illustration of a particular size of occluder 50 having been implanted into a patient's LAA. In this example, there is a relatively short distance between the position of the LAA and the patient's mitral valve MV. At least in part due to this relatively short distance, for the given desired size of lobe 58 to suitably anchor within and seal against the LAA, the correspondingly sized disc 56 may tend to protrude inferiorly so that it impinges or otherwise interferes with proper functioning of the mitral valve MV. In other words, the inferior portion of the disc 56 may tend to prevent the mitral valve leaflets from opening and closing in a normal manner, thus interrupting the proper functioning of the mitral valve MV. At least some embodiments described in greater detail below may help avoid the situation shown in Fig. 8, for example by being able to pair a smaller disc with the lobe 58 shown in Fig. 8, and/or by being able to pair a non-circular (*e.g*. oval or asymmetrically-shaped) disc with the lobe 58 of Fig. 8.

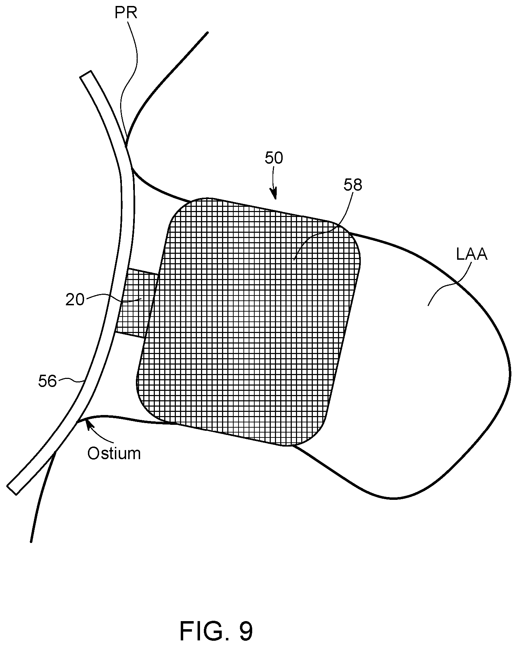

Fig. 9 is a side illustration of a particular size of occluder 50 having been implanted into a patient's LAA. In this example, the pulmonary ridge PR is relatively small for the size of the LAA. At least in part due to this relatively small pulmonary ridge PR, for the given desired size of lobe 58 to suitably anchor within and seal against the LAA, the correspondingly sized disc 56 may tend to protrude superiorly so that it extends too far into the left atrium, which may be undesirable for multiple reasons, including by possibly causing blockage of blood flowing from a pulmonary vein into the left atrium. At least some embodiments described in greater detail below may help avoid the situation shown in Fig. 9, for example by being able to pair a smaller disc with the lobe 58 shown in Fig. 9, and/or by being able to pair a non-circular (*e.g*. oval or asymmetrically-shaped) disc with the lobe 58 of Fig. 9.

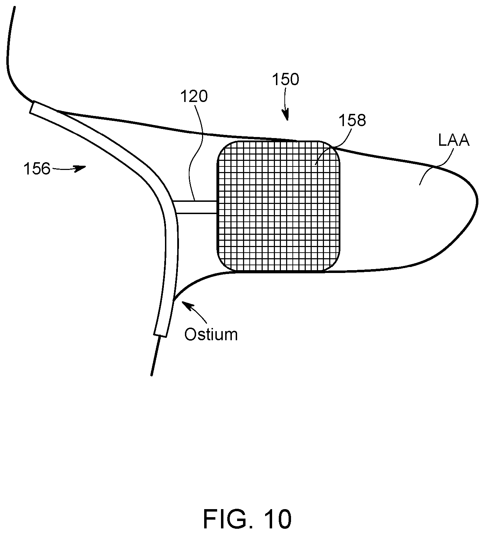

As described above, occluders in the prior art, such as occluder 50, are typically available in a limited range of sizes which include discs 56 that range in size from small to large, with corresponding lobes 58 that correspondingly range in size from small to large. One way to solve one or more of the potential problems shown and described in combination with Figs. 4-9 is to provide interchangeable components for an occluder 150 that is similar or identical to occluder 50, with the exception that different components of the occluder 150 can be mixed and matched. For example, Fig. 10 illustrates a LAA of a patient that includes a large ostium for the size of the LAA. With the standard sizes of occluder 50, an occluder 50 with a lobe 58 that is appropriately sized for the anchoring within and sealing against the LAA would be at risk of having a disc 56 that is too small to provide adequate coverage of the ostium. However, occluder 150 has an oversized disc 156 that has been separately connected to a relatively small lobe 158 via connector 120. In other words, although the structure of lobe 158 may be substantially identical to the structure of lobe 58, and although the structure of disc 156 may be substantially identical to the structure of disc 56, a "large" size disc 156 may be selected from a plurality of different sized discs and connected to the desired size of lobe 158 to appropriately occlude the particular anatomical example shown in Fig. 10. It should be understood that the occluder 50 described above is typically formed from braiding one or more nitinol wires into a metal fabric which is shape set to the desired shape, meaning that the occluder 50 from the lobe 58 to the disc 56, including the connecting member 20, is typically one integral member. Thus, providing a kit of (i) differently sized or shaped lobes, (ii) differently sized or shaped discs, and/or (iii) differently sized or shaped connectors is not necessarily a straightforward process that can be achieved with only simple modification of traditional manufacturing steps.

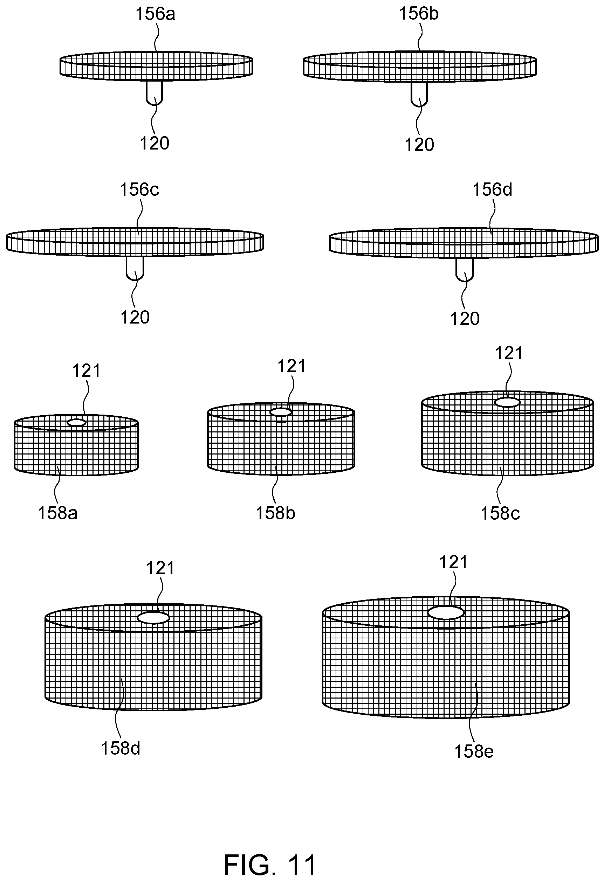

Fig. 11 illustrates one example of how a kit of interchangeable lobes and discs may be provided. In the particular example, four different diameter discs 156a, 156b, 156c, and 156d, with increasing diameters, may be provided along with five different diameter lobes 158a, 158b, 158c, 158d, 158e, with increasing diameters. In this particular example, each disc 156a-156d includes a similar or identical connection portion or waist 120 which can be coupled to a connector 121 of any one of the lobes 158a-158e to form an occluder. In this particular example, any of the four disc sizes 156a-d can be coupled to any of the five lobe sizes 158a-158e, such that only nine separate components need to be stocked to achieve a total of twenty different unique size combinations of an occluder. However, it should be understood that the kit of Fig. 11 is merely exemplary. For example, as noted above, occluder 50 may be provided in a total of eight sizes, with only eight unique size combinations. If the kit of Fig. 11 were instead provided with eight differently sized discs, each of which can be coupled to any of eight differently sized lobes, a total of sixteen components would provide sixty-four unique size combinations of the assembled occluder.

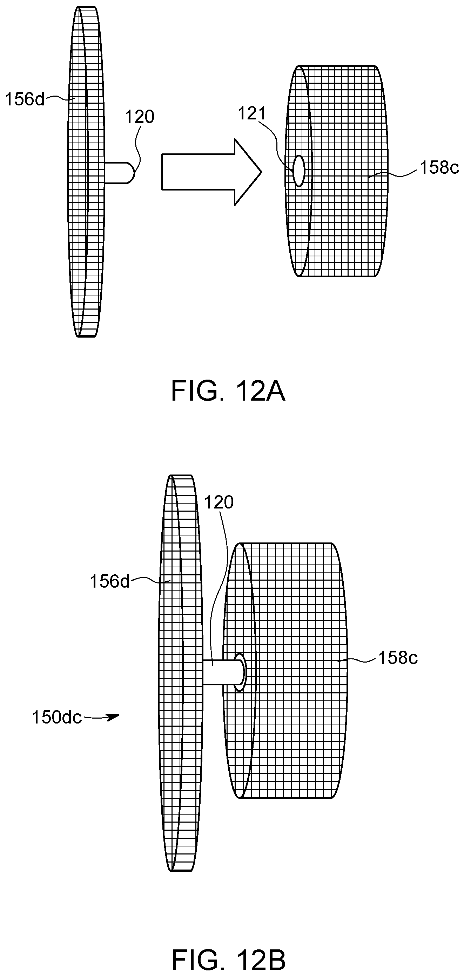

An exemplary use of the kit of Fig. 11 is now described. In some examples, the kit of Fig. 11 may be provided to a physician for use in an LAA occlusion procedure. The subject patient may be evaluated to measure the anatomy of the LAA, which may include the measurement of surrounding structures of the heart. For example, the patient's anatomy may be imaged by any one or more suitable modalities, including for example computed tomography ("CT"), echocardiography (*e.g*., transesophageal echocardiogram or "TEE"), and/or fluoroscopy. The anatomy, including for example the size and shape of the LAA, as well as relevant distances to the mitral valve and/or pulmonary ridge and/or pulmonary vein may be measured based on the imaging. Example measurements may include the depth of the LAA, the diameter of the LAA at one or more points in the LAA, the size and shape of the ostium of the LAA, the distance between the LAA ostium and the mitral valve, and the distance between the LAA ostium and the pulmonary ridge. With the relevant measurements complete, the physician would determine the desired size of the lobe 158 and the desired size of the disc 156, and select the two matching (or most appropriate) sized components from the kit. For example, as shown in Fig. 12A, the physician may determine that lobe 158c is the most suitable size for positioning within the LAA while disc 156d is the most appropriate size for covering the ostium of the LAA. Those two components may be selected from the kit, and the connector 120 of the disc 156d may be coupled to a corresponding connector 121 in the lobe 158c to form an occluder 150dc with a unique size combination of lobe and disc. This assembly may occur within the cath lab, for example at a sterile prep table, with a large number of size combinations of the occluder 150 being available despite only needing a relatively small number of individual components, which may be manageable both from a manufacturing/inventory perspective as well as an available shelf-space perspective within the cath lab. Once occluder 150dc is assembled, it may be attached to a delivery system and prepared for implantation similar to occluder 50. Compared to how occluder 50 may be delivered, the only different delivery step involved with occluder 150dc (beyond the assembly step) is that occluder 150dc may need to be actively attached (*e.g*. threaded or otherwise coupled) to the delivery device (*e.g*. delivery cable 108) by the end user, whereas occluder 50 may come pre-attached to the delivery device.

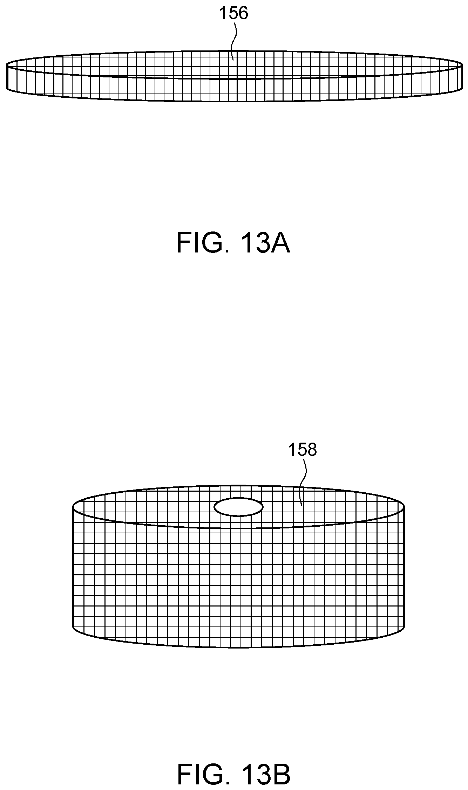

Figs. 13A-B illustrate exemplary individual disc 156 and lobe 158 components, respectively, of the kit of components shown in Fig. 11. It should be understood that in these embodiments, the disc 156 and lobe 158 are formed as braided mesh components, for example generally similar to the disc 56 and lobe 58 of occluder 50. For example, the same nitinol wire braided together to form occluder 50 may be used to form the discs 156 and the lobes 158 of the kit of interchangeable components. The discs 156 and lobes 158 may be formed in generally the same way as occluder 50 is formed. For example, occluder 50 may be formed by pulling braiding wires of nitinol together to form a braided mesh, and positioning the braided mesh in a double mold to form the disc 56 and the lobe 58 to the desired shape. This methodology is known in the art, and its relate techniques are described, for example, in U.S. Patent Nos. 6,368,339, 6,506,204, and 8,758,389, the disclosures of which are hereby incorporated by reference herein. However, rather than needing double molds to form the disc 56 and the lobe 58 simultaneously in occluder 50, the individual discs 156 and lobes 158 may only require a single forming mold per component.

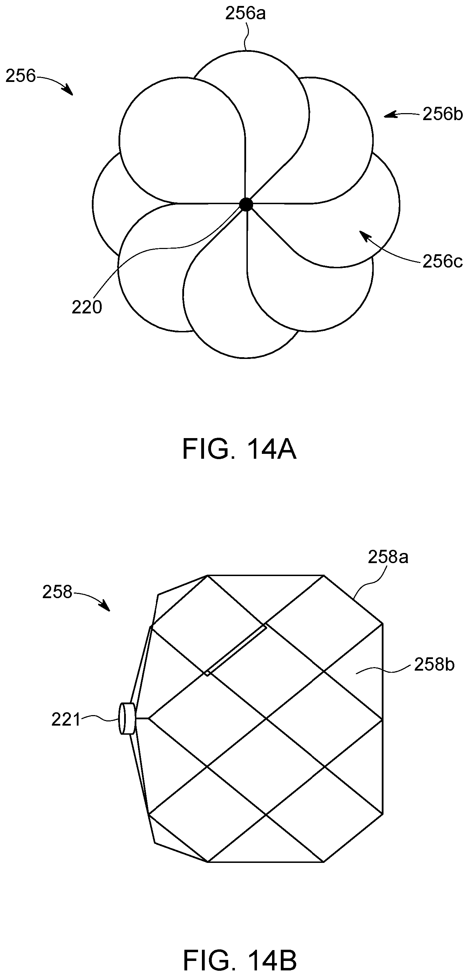

While much of the disclosure of this application has focused on interchangeable disc and lobe components formed of a braided mesh, it should be understood that the same concepts my be applied to other configurations of discs and lobes, for example discs and lobes that are cut (*e.g.* laser cut) from tubes or sheets of material (*e.g*. tubes or sheets of shape memory material such as nitinol) or otherwise formed via looping wires without needing to form a braided mesh. For example, Fig. 14A shows an alternate version of a disc 256 which may generally include wires 256a (e.g. nitinol wires) that are looped to form a plurality of overlapping petals 256b, which may include one or more occluding fabrics 256c spanning across the petal 256b to assist with sealing. It should be understood that if this type of disc 256 is used, it may be provided in a kit with a plurality of different sizes and/or shapes similar to the discs 156a-156d shown and described in relation to the kit of Fig. 11. Also similar to disc 156, disc 256 may include a connection portion 220 or waist so that any size disc 256 within the kit may be coupled to any desired size of lobe 258 included in the kit.

Fig. 14B shows one such exemplary lobe 258 that is not formed of a braided mesh. Rather, the particular example of lobe 258 shown in Fig. 14B is formed by laser cutting a tube of shape-memory material, in this case a tube of nitinol, and then shape setting it (*e.g.,* via heat treatment) into the desired shape of the lobe 258 shown in Fig. 14B. In some examples, the resulting lobe 258 may include a plurality of struts 258a that form cells 258b, which may be diamond-shaped or other similar shapes that assist with the lobe 258 collapsing to a small diameter for delivery and then re-expanding to the set shape during deployment into the LAA. As with disc 256, it should be understood that if this type of lobe 258 is used, it may be provided in a kit with a plurality of different sizes and/or shapes similar to the lobes 158a-158e shown and described in relation to the kit of Fig. 11. Also similar to lobe 158, lobe 258 may include a connector 221 that any size disc 256 within the kit may be coupled to any desired size of lobe 258 included in the kit. It should further be understood that different types of lobes and discs may be mixed and matched in a particular kit. For example, a set of braided mesh discs 156 may be provided with a set of laser-cut lobes 258, or a set of wire petal discs 256 may be provided with a set of braided mesh lobes 158, or any other suitable set of discs may be combined with any other suitable set of lobes. Further, lobe 258 may be provided with a sealing member, such as a fabric or polymer layer adhered, sutured, or otherwise attached to the lobe 258, and which may be provided on or in the proximal surface of the lobe 258 to provide and/or enhance sealing.

Referring briefly again to Figs. 12A-B, in some embodiments, the kits may include a total of two types of components. For example, connection portion 120 may be formed integrally with (or otherwise permanently attached to) the disc 156, with a free end of the connection portion 120 being coupled to a corresponding connector 121 in the lobe 158. In other examples, a connection portion similar to connection portion 120 show in Figs. 12A-B could be formed integrally with (or otherwise permanently attached to) the lobe 158, with a free end of that connection portion being coupled to a corresponding connector in the disc 156. In either scenario, a separate set of connector components is not needed with the kit.

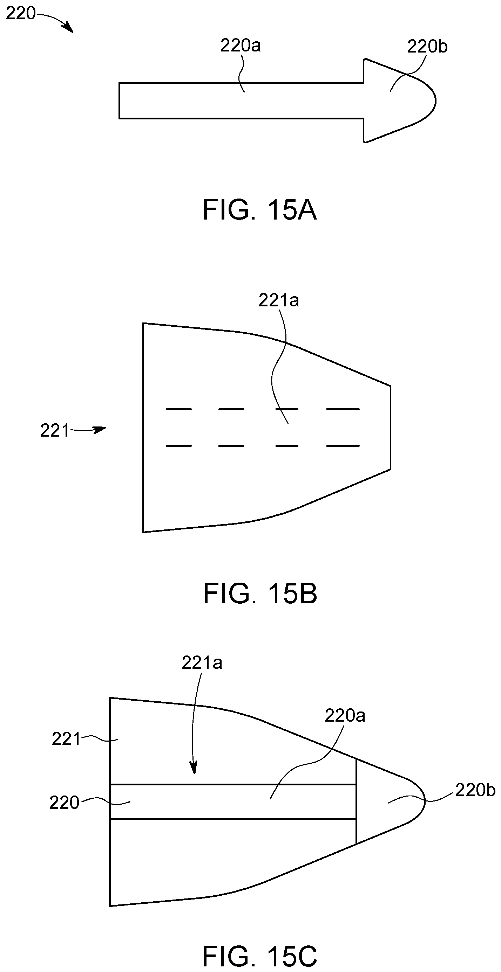

If the connection portion 120 is being provided as integral with, or otherwise permanently pre-attached to either the disc 156 or the lobe 158, the connection portion may take any suitable form. For example, Fig. 15A shows a first connector 220 that may be provided permanently coupled to one of the disc 156 and the lobe 158, while Fig. 15B shows a second connector 221 that may be provided permanently coupled to the other of the disc 156 and the lobe 158. In this particular example, the first connector 220 may include a generally cylindrical shaft 220a that extends from a first end and terminates in an enlarged head 220b at a second end, which may have a generally conical shape, and which may be slightly compressible. The first end of the shaft 220a may be permanently connected to one of the disc 156 or the lobe 158 so that the enlarged head 220b is at a free end. The second connector 221 may be generally frustoconical, extending from a large diameter first end to a smaller diameter second end, and the second end may be permanently connected to the other of the disc 156 or the lobe 158 so that the large diameter first end is at a free end. The second connector 221 may define a channel 221a extending therethrough, the channel having a diameter that is about equal to (or slightly larger than) that of the shaft 220a, but which is smaller than that of the enlarged head 220b. In order to connect the disc 156 and the lobe 158, the first connector 220 may be pushed through the channel 221a of the second connector, causing the enlarged head 220b to temporarily compress until it emerges through the second fixed end of the second connector 221, at which point the enlarged head 220b may decompress to its normal enlarged diameter, preventing the two connectors 220, 221 from being disconnected as shown in Fig. 15C. It should be understood that, if the second smaller diameter end of the second connector 221 is permanently coupled to the disc 156 or the lobe 158, a small recess may be provided within the disc 156 or the lobe 158 to accommodate the enlarged head 220b. It should be understood that the push-to-lock configuration shown and described in connection with Figs. 15A-C is just one example of many types of push-to-lock devices that may be suitable for use as connectors 120, 121, and thus connectors 120, 121 should not be considered limited to the particular example of Figs. 15A-C.

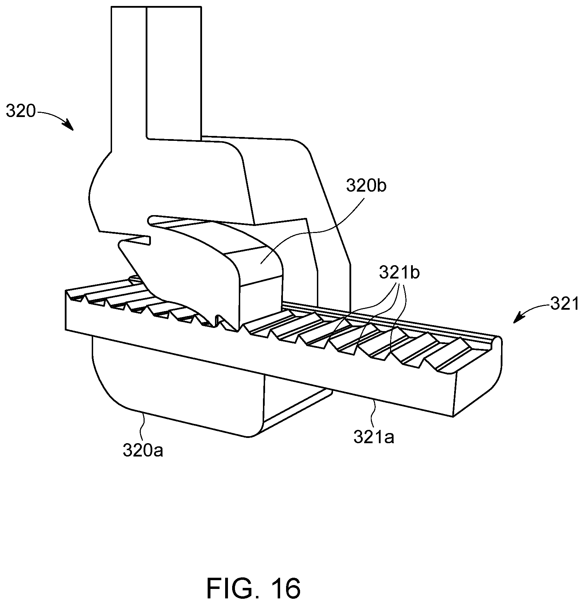

The connectors 220, 221 described in connection with Figs. 15A-C may be generally fixed length connectors. In other embodiments, variable length connectors may be provided, for example fixed to the disc 156 and/or the lobe 158 so that additional components in the kit are not needed. For example, Fig. 16 shows a connection mechanism in an assembled state that includes a first connector 320 assembled to a second connector 321, which may be an adjustable-length connector with similarities to how a zip tie or cable tie functions. For example, the first connector 320 may have a body 320a that, along with a ratchet pawl 320b, forms a pathway through which the second connector 321 may slide. The second connector 321 may also include a body 321a, with one face of the body 321a including a plurality of serrations (or teeth) 321b that engage the ratchet pawl 320b in a one-way configuration. The first connector 320 may be fixed to one of the disc 156 or the lobe 158, and the second connector 321 may be fixed to the other of the disc 156 or the lobe 158, and the second connector 321 may be slid through the first connector 320 to couple the two connectors via the engagement between ratchet pawl 320b and serrations 321b. Due to the one-way configuration, the two connectors will not decouple from each other, but depending on how far the second connector 321 is passed through the first connector 320, the total length of the assembled connectors may change. With this configuration, the physician may assemble the disc 156 to the lobe 158 in the cath lab to any desired length, allowing for more customization without needing additional components. Although this ratchet mechanism is one example of an adjustable-length connection system, other suitable connection types may include a threaded connection (*e.g*. male threads on one connector, female threads in a second connector, similar to a screw) that allows for secure connection between the components with the amount that the first connector is threaded into the second connector determining the total length of the connection assembly. Another suitable variable length connection mechanism may be a suture or wire type in which a suture-type of material is tied to both the disc 156 and the lobe 158, with the length of the suture-type material after being tied determining the length of the connector. It should be understood that these structures are merely exemplary, and various other suitable mechanisms may allow for a variable-length connection between the disc 156 and the lobe 158 to help optimize and/or customize the length between the disc 156 and the lobe 158, as well as the resulting tension between the disc 156 and the lobe 158 upon implantation (with shorter connector lengths corresponding to greater tensions).

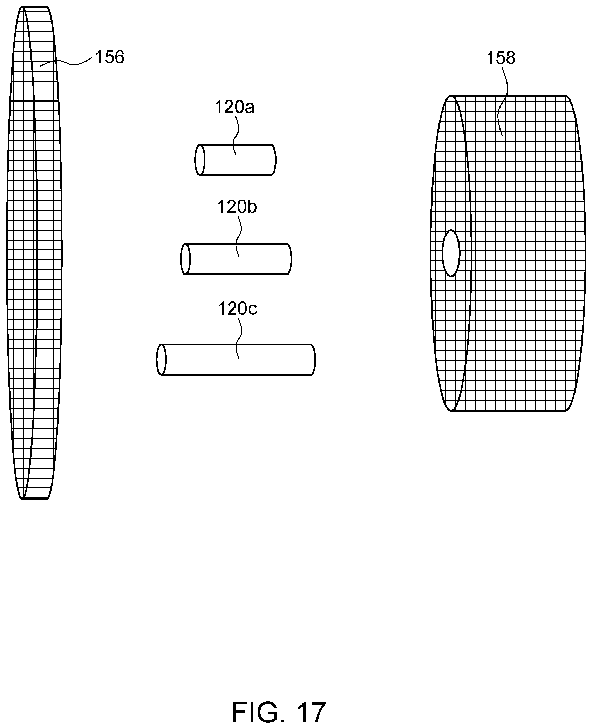

Although the connectors may be provided to the end user as part of the disc 156 or lobe 158 (*e.g.,* formed integrally with the components or pre-connected to the components), in other examples, a set of separate connectors may be provided as part of the kit to provide for even more possible custom configurations of occluder 150. For example Fig. 17 shows an example in which, in addition to a plurality of differently sized discs 156 and lobes 158, the kit includes a plurality of different size connectors 120a, 120b, 120c. In this particular example, a short connector 120a, a medium connector 120b, and a long connector 120c are each provided as part of the kit, but it should be understood that two, or more than three connectors of different sizes and/or lengths may be provided as part of the kit of differently sized lobes and discs. Similar to providing a variable length connector, providing a plurality of differently sized connectors 120a, 120b, 120c may help a physician customize the occluder 150 to account for the patient's anatomy and to optimize tension between the disc 156 and the lobe 158 after deployment. The connectors 120a, 120b, 120c may take many suitable forms, some of which are described in more detail below.

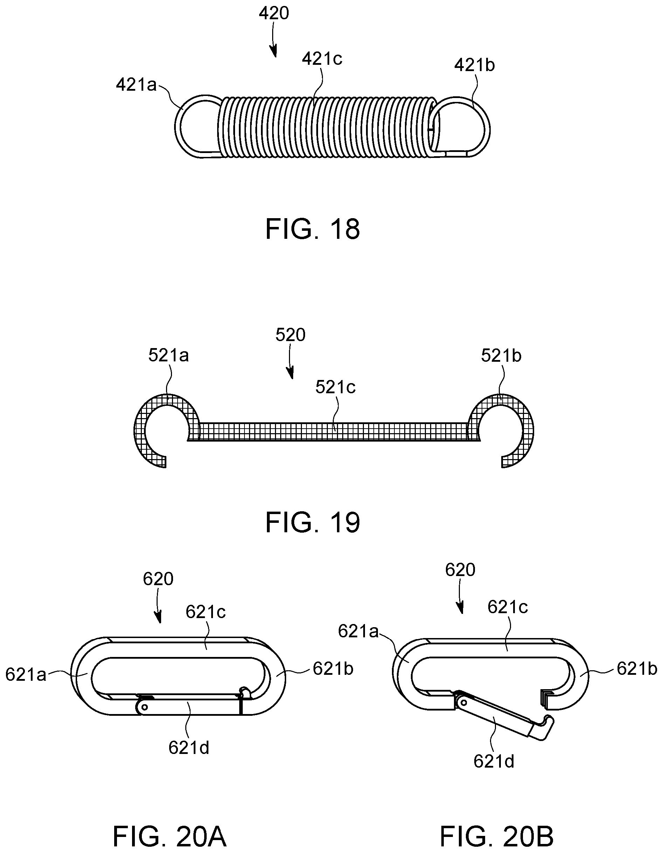

Fig. 18 shows one example of a connector 420 that may be used as the differently sized connectors 120a, 120b, 120c of Fig. 17. In the illustrated embodiment, connector 420 has a spring configuration including a first connecting end 421a and a second connecting end 421b, which may for example be wire loops, and a main spring body 421c extending therebetween. A plurality of connectors 420, for example with different lengths for the main spring body 421c, may be provided with a kit of differently sized discs 156 and lobes 158. In use, the physician may choose the desired size connector 420 (as well as the desired size of disc 156 and lobe 158), and connect first connecting end 421a to the disc 156 and second connecting end 421b to the lobe 158, with the main spring body 421c helping to maintain tension and connection between the disc 156 and the lobe 158 upon deployment of the assembled occluder 150.

Fig. 19 shows another example of a connector 520 that may be used as the differently sized connectors 120a, 120b, 120c of Fig. 17 (*e.g.,* as an alternate to spring connector 420). In the illustrated embodiment, connector 520 has a suture configuration including a first connecting end 521a and a second connecting end 521b, which may for example be ends of a suture, and a main suture body 521c extending therebetween. A plurality of connectors 520, for example with different lengths for the main suture body 521c, may be provided with a kit of differently sized discs 156 and lobes 158. In use, the physician may choose the desired size connector 520 (as well as the desired size of disc 156 and lobe 158), and connect first connecting end 521a to the disc 156 (*e.g.* by tying a knot) and second connecting end 521b to the lobe 158 (*e.g.,* by tying a knot), with the main suture body 521c helping to maintain connection between the disc 156 and the lobe 158, as well as transfer tension between the disc 156 and the lobe 158, upon deployment of the assembled occluder 150.

Figs. 20A-B show another example of a connector 620 that may be used as the differently sized connectors 120a, 120b, 120c of Fig. 17 (*e.g.,* as an alternate to spring connector 420 or suture connector 520). In the illustrated embodiment, connector 620 has a clasp configuration including a first curved end 621a and a second curved end 621b, and a main body 621c extending therebetween. The connector 620 may also include a moving portion 621d that may be moved between a closed condition, shown in Fig. 20A, and an open condition, shown in Fig. 20B. In the closed condition of Fig. 20A, the clasp connector 620 forms a closed boundary, while in the open condition of Fig. 20B, the clasp connector 620 does not form a closed boundary. In some examples, the moving portion 621d is biased, for example via a spring, to the closed condition of Fig. 20A. A plurality of connectors 620, for example with different lengths for the main body 621c and the moving portion 621d, may be provided with a kit of differently sized discs 156 and lobes 158. In use, the physician may choose the desired size connector 620 (as well as the desired size of disc 156 and lobe 158), transition the moving portion 621d to the open condition, and hook the first curved end 621a to the disc 156 (*e.g.* by hooking it around a loop structure of the disc 156) and hook the second curved end 621b to the lobe 158 (*e.g.,* by hooking it around a loop structure of the disc 156). Once connected, the user may move the moving structure 621d back to the closed condition (for example by removing any applied force to the moving structure 621d) with the main clasp body 621d (as well as the closed moving portion 621a) helping to maintain connection between the disc 156 and the lobe 158, as well as transfer tension between the disc 156 and the lobe 158, upon deployment of the assembled occluder 150.

Connectors 420, 520, and 620 are merely exemplary of types of connectors that may be provided in a plurality of sizes (or lengths) as part of a kit, but it should be understood that various other types of connector structures may be suitable beyond these specific examples. For example, a paperclip style, spring hook style, or key-ring style connector may be provided in various different sizes, with the selected length connector being coupled to the disc 156 and the lobe 158 in the same way a paperclip or spring hook or key-ring is connected to a structure. In another example, a wire or cable that terminates in two threaded ends may be used (with multiple lengths for the main cable portion), with each threaded end being configured to screw or thread into correspondingly threaded receivers in the disc 156 and the lobe 158. In a further example, a rivet-type connector may be used, for example in which a tube has opposite ends that are positioned within rivet connectors on the disc 156 and the lobe 158 which are subsequently clamped or crimped over the ends of the tube to couple the disc 156 to the lobe 158 with the desired length of connection tubing selected from the kit. In still another example, a buckle clip connector may be used, for example in which a male buckle has a first end coupled to the disc 156 and a female buckle (or receiver) has a first end coupled to the lobe 158, with the free ends of the male and female buckles being connected or clipped together to couple the disc 156 to the lobe 158. Other types of connectors may similarly be suitable.

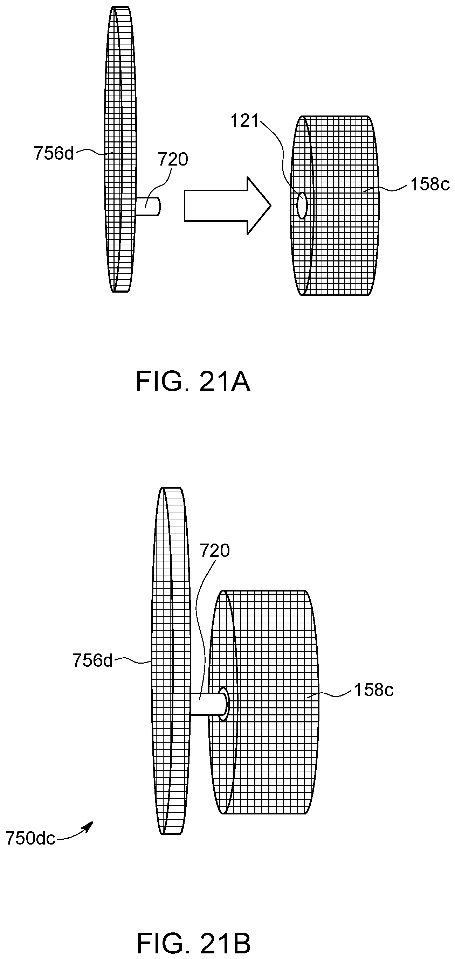

Although the embodiments above have generally focused on kits that include different sizes of discs 156 and lobes 158 having a typical circular or cylindrical shape, kits may also include one or more differently shaped components. For example, the kit of Fig. 11 (or a similar kit) includes discs 156 in which the connecting portion 120 is generally aligned with a longitudinal center of the disc 156 such that the resulting occluder (when disc 156 is coupled with lobe 158) is substantially rotationally symmetric. In another embodiment, one or more differently shaped discs may be provided. For example, as shown in Figs. 21A-B, an eccentric disc 756d (which may represent the largest of four eccentric discs included in a kit similar to that shown in Fig. 11) may be selected from the kit and coupled to lobe 158c to form an occluder 750dc. The eccentric disc 756d may have an oval or elliptical shape, with the connecting member 720 being positioned away from the center of the disc 756d. In some examples, the connecting member 720 may instead be positioned at the center of the oval-shaped disc 756d. In some examples, the disc 756d may still be circular like disc 156d, but the connector 720 is offset so that, when the disc and lobe are coupled, the resulting occluder is not rotationally symmetric. Other shapes of discs beyond the circular and oval discs described above may also be provided within a kit to provide a significant number of customizable occluder configurations without having an unmanageable number of components within the kit. The particular occluder 750dc of Fig. 21B may be helpful in situations in which the patient's anatomy calls for the disc 756d to bias to one side more than the other, such as at bifurcated LAA lobes or large pulmonary ridges where there is not much space between the mitral valve and the LAA. For example, occluder 750dc may be well suited to the anatomy shown in Fig. 8 to provide good coverage of the pulmonary ridge PR without interfering with the mitral valve MV. It should be understood that, if different shapes of the lobe and/or disc are provided with a kit, they may also be provided in different sizes, as well as with multiple lengths of connectors, with variable length connectors, or with fixed connectors, all as described above.

The present application refers to a "kit" that may be used to assemble an occluder. It should be understood that the term "kit" may include a pre-assembled or pre-organized kit. For example, a "kit" may include a collection of items offered in a pre-packaged container that is provided to the end user in a pre-organized or pre-assembled manner. However, the term "kit" is not limited to pre-assembled or pre-organized groups of items that are packaged in a container as a single unit for sale. For example, a manufacturer may offer individual sizes and/or shapes of (i) proximal discs; (ii) distal lobes; and/or (iii) connectors as individual units that a physician or other user may order or purchase individually, based on the user's experience, for future assembly into an occluder and implantation into a patient. Even though these items are acquired or purchased as individual units, the group of individually acquired or purchased units (even if acquired or purchased at disparate times) may still constitute a "kit" as the term is used herein. In other words, the term "kit" as used herein - unless explicitly stated otherwise - is intended to refer to a grouping of items that may be assembled together to form an occluder suitable for implantation into a patient, regardless of how the items are grouped (*e.g*. whether in an organized, pre-assembled kit, or whether the items are obtained individually at different time points). For example, a kit may include a plurality of proximal discs and distal lobes that are all packaged in a single container, or a plurality of proximal discs packaged in a first container and a plurality of distal lobes packaged in a second container, or a plurality of proximal discs packaged in a plurality of first containers and a plurality of distal lobes packaged in a plurality of second containers.

The present application also refers to occluders that may be formed of "interchangeable" components such as an interchangeable proximal disc and an interchangeable distal lobe. As used herein, "interchangeable" may be used synonymously with "modular." For example, a modular proximal disc may be thought of as a proximal disc that is specifically designed for use with more than one specific size and/or shape of distal lobe. Similarly, a modular distal lobe may be thought of as a distal lobe that is specifically designed for use with more than one specific size and/or shape of a proximal disc. Thus, in this context, a proximal disc that is formed separately and later coupled to a distal lobe is not modular unless the proximal disc is specifically configured to be able to be coupled to distal lobes of different sizes and/or shapes. Similarly, in this context, a distal lobe that is formed separately and later coupled to the proximal disc is not modular unless the distal lobe is specifically configured to be able to be coupled to proximal discs of different sizes and/or shapes.

As explained above, the modular discs described herein and the modular lobes described herein can be formed having different base designs, such that disc having a first base design (*e.g.* braided mesh design, laser-cut stent design, polymer design, bioabsorbable design, *etc*.) can be paired with a lobe having a second base design (*e.g*. braided mesh design, laser-cut stent design, polymer design, bioabsorbable design, *etc*.) that is different from the first base design. This may apply to any of the embodiments described above. For example, a modular braided mesh disc having a desired size and shape may be coupled to a modular laser-cut stent style lobe, which may provide strong anchoring within the LAA (due to a relatively high stiffness or relatively low flexibility of the laser-cut stent style lobe) while providing high conformability to the tissue forming the ostium (due to a relatively low stiffness or relatively high flexibility of the braided mesh style disc). The base designs of the lobe and the disc can be mixed and matched to obtain different benefits for the different structures as desired. In other examples, both the lobe and the disc may formed with a braided mesh design, but the lobe may have a more dense mesh (*e.g.* by using more braided wires and/or braid wires with larger diameters), which may provide for enhanced anchoring, compared to the disc having a lower density mesh, which may provide for more conformability to seal against the tissue forming the ostium of the LAA. In another example, the disc may be provided with a polymer base design, which may provide even more conformability (*e.g*. lower stiffness or higher flexibility) compared to the braided mesh option described directly above, with the lobe still being formed having a stiffer or less flexible configurations (*e.g*. as a laser-cut stent). Still other base designs may be utilized. For, example, the disc may be formed of a bioabsorbable material, with the lobe being formed of a non-bioabsorbable material (although in other embodiments the opposite configuration may be used). With this configuration, the disc may be absorbed by the body over time, eliminating potential sites for metal-to-tissue irritation around the ostium of the LAA, or reducing material available for blood stagnation. In such embodiments, by the time the disc and/or the lobe is absorbed by the body, tissue ingrowth and/or blood clotting will have already occurred in the LAA so that the LAA remains sealed after the disc and/or lobe is absorbed.

The paragraph above addresses modularity in which the lobe and the disc have different base designs. Other examples of relevant base designs that can be mixed and matched include base designs that differ due to different processing. For example, these process-related base designs may include coating, polishing, and/or surface treatment. As one particular example of this, the modular lobe may be processed (whether formed as a braided mesh, a laser-cut stent style, or another style) to have a higher surface roughness than that of the disc. Higher surface roughness may be achieved by various methods, such as by forming indentations on the lobe, shot peening the lobe, abrading the lobe with a wire wheel, *etc.* By providing the lobe with a higher surface roughness, the anchoring functionality of the lobe may be enhanced, and a lobe with a higher surface roughness may be provided with a modular disc having a lower surface roughness. In another example, the modular disc may be electropolished to improve fatigue and reduce leaching of nickel (*e.g.* if the disc is formed of a nickel alloy). Similarly, the modular disc may be provided with a pro-thrombotic treatment, without any similar treatment applied to the modular lobe that is selected for coupling to the modular disc. Another process that may be performed on only one of the modular disc or the modular lobe is an electrospinning process to coat the modular component in an occlusive material, instead of manually attaching an occlusive fabric to the component. As should be clear, any combination of desired base designs (including both structure-based and process-based designs) may be applied to one or more modular lobes, with any combination of desired base designs being applied to one or more modular discs, with the particular final combination of lobe and disc being selected per the manufacturer's or user's overall desire. In other words, the manufacturer may select the particular properties for the base designs of the modular disc and the modular lobes, and those modular pieces may be provided in different sizes with the end user selecting a particular combination of sizes. However, in other examples, the modular discs and the modular lobes themselves may be provided in a variety of different base designs that the user may select, along with desired sizes, for assembly into an occluder. It should also be clear that, although particular combinations of base designs are explicitly addressed above, any desired combination of base designs is within the scope of this disclosure.

Much of the disclosure above is provided in the context of an end user (*e.g.* a physician) manually forming or manufacturing an occluder by coupling a modular disc having a first base design, size, and/or shape with a modular lobe having a second base design, size, and/or shape (optionally via a third modular connector having a desired size or length). However, it should be understood that this manufacturing step (*i.e.* the coupling of the already-formed modular disc and already-formed modular lobe) may be performed elsewhere. For example, the physician may determine the specific modular lobe and specific modular disc (and optionally a specific modular connector) that would be best suited for the patient, and may order an occluder from a provider, where the provider is the one that couples the modular components together to form the occluder. In this example, the end user may not need to store any kits of components on hand, rather ordering from the provider what amounts to a custom (or at least partially custom) occluder. From the perspective of the provider, the provider may receive selections of a particular modular lobe and a particular modular disc (and optionally a particular modular connector), assemble the occluder based on these selections, and then provide the final assembled occluder to the end user. In still other examples, instead of the user actually selecting a particular combination of modular components and ordering an occluder configured using the selected modular components, the end user may instead provide relevant data, such as anatomical measurements of the patient, to the provider who may select optimized modular components based on such measurements and assemble the occluder by coupling the optimized modular components together. Similarly, from the perspective of the provider, the provider may receive from the end user one or more data sets, such as anatomical measurements of the patient, and select optimized modular components based on such measurements, assemble the occluder by coupling the optimized modular components together, and then provide the assembled occluder to the end user.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A kit for assembling a collapsible and expandable occluder (150dc) for occluding a left atrial appendage (LAA), the kit comprising:
a plurality of proximal discs (156a-d) each configured to cover an ostium of the LAA in an implanted condition of the occluder (150dc), each of the plurality of proximal discs (156a-d) having a different diameter in an expanded condition than other ones of the plurality of proximal discs (156a-d); and
a plurality of distal lobes (158a-e) each configured to be received within a cavity of the LAA in the implanted condition of the occluder (150dc), each of the plurality of distal lobes (158a-e) having a different diameter in an expanded condition than other ones of the plurality of distal lobes (158a-e);
wherein each of the plurality of proximal discs (156a-d) is configured to couple to any one of the plurality of distal lobes (158a-e) to form the occluder (150dc).

2. The kit of claim 1, wherein each of the plurality of proximal discs (156a-d) is formed of a braided mesh and each of the plurality of distal lobes (158a-e) is formed as a braided mesh.

3. The kit of claim 1, wherein each of the plurality of proximal discs (256) is formed of overlapping wire petals (256b) and each of the plurality of distal lobes (258) is formed of a laser cut tube of shape memory material.

4. The kit of any of the preceding claims, wherein each of the plurality of proximal discs (156a-d) has a corresponding first connection portion (120) coupled thereto, and each of the plurality of distal lobes (158a-e) has a corresponding second connection portion (121) coupled thereto, each of the first connection portions (120) being configured to couple to any one of the second connection portions (121).

5. The kit of claim 4, wherein each first connection portion (220) includes a shaft (220a) and an enlarged head (220b), and each second connection portion (221) includes a receiver defining a channel (221a) therethrough, the shaft (220a) and enlarged head (220b) configured to pass through the channel (221a) in a first direction, the enlarged head (220b) being compressible and having a diameter that is larger than a diameter of the channel (221a) when the enlarged head (220b) is in an uncompressed condition.

6. The kit of claim 4, wherein each of the first connection portions (120) is configured to couple to any one of the second connection portions (121) to form an assembled connector, the assembled connector having a variable length.

7. The kit of claim 6, wherein each first connection portion (320) includes a ratchet pawl (320b), and each second connection portion (321) includes a plurality of serrations (321b) so that each second connection portion (321) is configured to pass through any one of the first connection portions (320) in a first direction, but not in a second direction opposite the first direction.

8. The kit of any of the preceding claims, further comprising a plurality of connectors (120a-c) configured to couple any of the plurality of proximal discs (156a-d) to any one of the plurality of distal lobes (158a-e), each of the plurality of connectors (120a-c) having a different length.

9. The kit of claim 8, wherein each of the plurality of connectors (120a-c) is a spring connector (420) having a first end (421a), a second end (421b), and a main spring body (421c) between the first end (421a) and the second end (421b), the main spring body (421c) of each of the plurality of connectors (120a-c) having a different length.

10. The kit of claim 1, wherein the plurality of proximal discs (156a-d) are each substantially circular with a connection portion (120) extending from a radial center thereof, and the kit further includes at least one eccentric proximal disc (756d) having a connection portion (720) extending therefrom, the connection portion (720) of the at least one eccentric proximal disc (756d) being offset from a radial center of the at least one eccentric proximal disc (756d).

11. The kit of any of the preceding claims, wherein the plurality of proximal discs (156a-d) include at least one circular proximal disc and at least one oval-shaped proximal disc.

12. The kit of any of the preceding claims, further comprising a single container that includes therein the plurality of proximal discs (156a-d) and the plurality of distal lobes (158a-e).

13. The kit of any of claims **1-11,** further comprising a first container that includes therein the plurality of proximal discs (156a-d), and a second container that includes therein the plurality of distal lobes (158a-e).

14. The kit of any of claims **1-11,** further comprising a plurality of first containers that include therein the plurality of proximal discs (156a-d), and a plurality of second containers that include therein the plurality of distal lobes (158a-e).

15. The kit of any of the preceding claims, wherein the plurality of proximal discs (156a-d) are formed with a first base design, and the plurality of distal lobes (158a-e) are formed with a second base design different from the first base design so that the plurality of proximal discs (156a-d) are more flexible than the plurality of distal lobes (158a-e), and the plurality of distal lobes (158a-e) are stiffer than the plurality of proximal discs (156a-d).
